(19) Europäisches Patentamt
European Patent Office
Office européen des brevets

(11) **EP 4 101 442 B1**

(12) **EUROPEAN PATENT SPECIFICATION**

(45) Date of publication and mention
of the grant of the patent:
**03.09.2025 Bulletin 2025/36**

(21) Application number: **21382522.7**

(22) Date of filing: **11.06.2021**

(51) International Patent Classification (IPC):
*A61K 9/16* *(2006.01)*    *A61K 8/35* *(2006.01)*
*A61K 9/20* *(2006.01)*    *A61K 9/48* *(2006.01)*
*A61K 31/122* *(2006.01)*    *A61P 43/00* *(2006.01)*
*A61Q 19/08* *(2006.01)*

(52) Cooperative Patent Classification (CPC):
**A61K 9/1688; A61K 8/355; A61K 9/2009;
A61K 9/2054; A61K 9/4858; A61K 31/122;
A61P 43/00; A61Q 19/08**

(54) **PROCESS FOR PREPARING COMPACTED COENZYME Q10 PARTICLES OF HIGH DENSITY AND IMPROVED FLOWABILITY**

VERFAHREN ZUR HERSTELLUNG VON VERDICHTETEN COENZYM-Q10-PARTIKELN MIT HOHER DICHTE UND VERBESSERTER FLIESSFÄHIGKEIT

PROCÉDÉ DE PRÉPARATION DE PARTICULES COMPACTÉES DE COENZYME Q10 À HAUTE DENSITÉ ET À FLUIDITÉ AMÉLIORÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**14.12.2022 Bulletin 2022/50**

(73) Proprietor: **Tradichem Industrial Services, S.L.
28108 Alcobendas (ES)**

(72) Inventors:
• **MARAÑÓN MAROTO, José Ángel
28240 HOYO DE MANZANARES (ES)**
• **MORENO PUENTE, Patricia
28007 MADRID (ES)**
• **LOZANO MARTIN, Cristina
28251 RIVAS-VACIAMADRID (ES)**
• **MANCHA AVELLÁN, María
28028 MADRID (ES)**
• **MUÑOZ PLAZA, David
28037 MADRID (ES)**

(74) Representative: **Sugrañes, S.L.P.
Calle Provenza 304
08008 Barcelona (ES)**

(56) References cited:
**EP-A1- 2 415 467**    **EP-A2- 0 083 108**
**US-A- 5 378 461**    **US-A1- 2010 004 473**

Remarks:
The file contains technical information submitted after the application was filed and not included in this specification

EP 4 101 442 B1

**Description**

Technical field

The present invention relates to a process for preparing compacted high density coenzyme Q10 particles, which have improved flowability and handling properties and are therefore advantageous for manufacturing pharmaceutical compositions comprising coenzyme Q10 as active ingredient.

Technical background

Coenzyme Q10 (CoQ10) is an endogenous, vitamin-like benzoquinone, which has an important role in electron transport in mitochondrial membranes for the production of ATP and which also functions as an endogenous antioxidant.
It has been disclosed that some individuals suffer from CoQ10 deficiency, for example, due to genetic or aging factors, carcinogenesis processes or caused by statin treatment. In this connection, there are numerous diseases associated with CoQ10 deficiency which can benefit from the administration of coenzyme Q10, for example, cardiovascular diseases, neurodegenerative diseases, cancer, diabetes mellitus or male infertility, among others (Garrido-Maraver et al., Coenzyme Q10 Therapy, Mol. Syndromol., 2014, 187-197).
Furthermore, due to its anti-oxidant properties, CoQ10 is also frequently used in topical formulations in the cosmetic field, for reducing photoaging effects, for wrinkle reduction and for increasing epidermal turnover (Hoppe et al., Coenzyme Q10, a cutaneous antioxidant and energizer, BioFactors, 1999, 9, 371-378).
Therefore, compositions comprising CoQ10 as active ingredient are widely available, either as dietetic supplement, generally in form of capsules, softgels or tablets, or as topical formulation.
However, the preparation of formulations comprising CoQ10 is challenging due to the unfavourable physicochemical characteristics of this substance. Firstly, CoQ10 is virtually insoluble in water, which makes it difficult the preparation of certain type of formulations and also results in poor bioavailability of the oral dosage forms. The problem of the poor water solubility and bioavailability of CoQ10 has been thoroughly approached in the art, and several solutions have been proposed, for example, size reduction, solid dispersions, cyclodextrin complexation, oily formulations in softgel capsules, or self-emulsifying drug delivery system (SEDDS) (Beg et al., Bioavailability enhancement of Coenzyme Q10: an extensive review of patents, Recent patents on Drug Delivery & Formulation 2010, 4, 245-257.
Other important drawbacks for preparing coenzyme Q10 formulations are related to the instability and poor rheology of this substance. Indeed, coenzyme Q10 is a fine yellow to orange crystalline powder which is unstable and vulnerable to heat, light and oxygen. It decomposes and darkens when exposed to light, has low melting point, around 48 °C, is highly hygroscopic and has poor flowability. Therefore, CoQ10 is difficult to be dosed accurately and pressed into tablets, and is difficult to handle as it easily adheres to machinery surfaces (Arenas-Jal et al., Coenzyme Q10 supplementation: Efficacy, safety, and formulation challenges, Compr. Rev. Food Sci. Food Saf., 2020, 19, 574-594).
Some solutions to these problems have been suggested in the art. The U.S. patent application US-A-2010/0004473 discloses a method for producing CoQ10 particles with improved handling and flowability. This method comprises mixing CoQ10 with a poor solvent, for example, aqueous ethanol solution or aqueous surfactant solution, previously heated at a temperature which is higher than the melting point of CoQ10, so that CoQ10 becomes melt and dispersed in the form of oil droplets, then the dispersion is cooled to below the solidification temperature of CoQ10, and the solid particles are then filtrated.
The European patent application EP-A-2415467 also discloses a method for producing CoQ10 particles with improved fluidity and handling properties. The method comprises compression molding of CoQ10, for example using a roller-type compression machine, under specific pressure and temperature conditions, and then grinding the compressed fragment obtained. The temperature of CoQ10 during the compression must be in the range 35-52 °C, and it is specifically stated that when the product temperature is lower than 35 °C a satisfactory compression molded product is not obtainable. Furthermore, for obtaining the best flowable product, having a low angle of repose of about 7 to 18 degrees, an additional step of heat treatment of the obtained powder is required, at a temperature in the range 30-52 °C. This heat treatment may cause that some particles fuse with each other, so an additional grinding step may be necessary.
Despite the few disclosures available in the state of the art, there is still the need of simple and efficient methods for producing CoQ10 particles of improved flowability, which would allow a better handling of this substance during the manufacture of pharmaceutical compositions and would also avoid substance losses during the manufacturing process of said compositions.

Object of the invention

The object of the present invention is a process for the preparation of compacted coenzyme Q10.

Description of the drawings

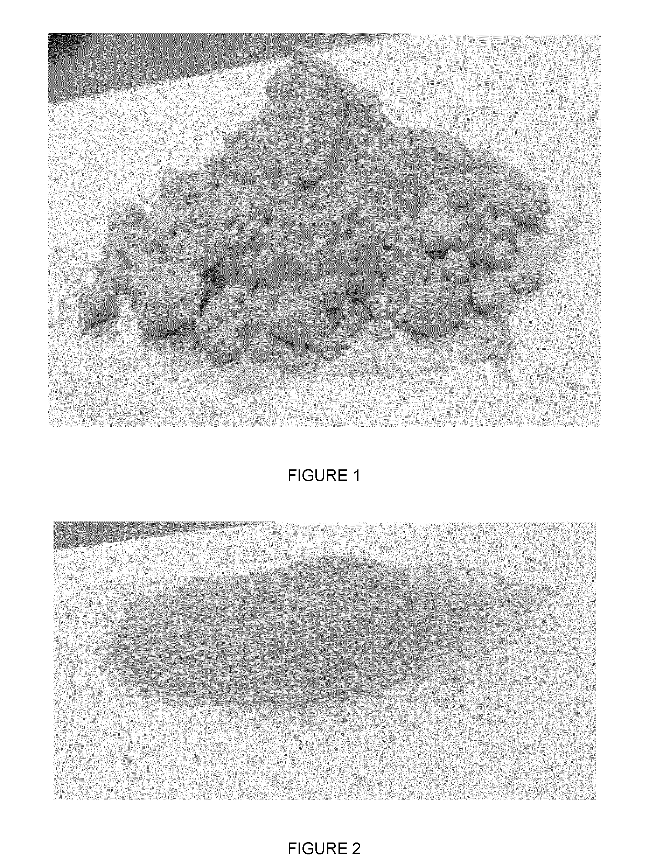

Figure 1 shows a picture of non-compacted CoQ10 starting material used in Example 1 and Figure 2 shows a picture of the compacted CoQ10 obtained in Example1.

Detailed description of the invention

The object of the present invention is a process for preparing compacted coenzyme Q10 comprising roller compaction of coenzyme Q10 at a temperature comprised between 10 °C and 25 °C.

The authors of the present invention found that, when coenzyme Q10 is subjected to roller compaction at the specified temperature, the compacted coenzyme Q10 particles thus obtained show improved rheological properties, so the product is flowable, is not sticky and does not form agglomerates and is, therefore, optimal for being handled and for manufacturing pharmaceutical compositions. Surprisingly, the authors of the present invention found out that when the temperature of the product is kept low enough during this compacting process, namely, at a maximum of 25 °C, the flowability properties of the compacted substance are particularly improved. Furthermore, the compacted coenzyme Q10 particles prepared with the present process are substantially pure and do not contain any excipient, which make them very versatile and suitable for preparing any type of composition.

Along the present description, as well as in the claims, singular expressions, generally preceded by the articles "a", "an" or "the", are intended to include the plural forms as well, unless the context clearly indicates otherwise.

The terms "about" or "approximately" referred to amounts, as used herein, are meant to include the exact amount and also a certain deviation around the stated amount, namely of $\pm 5\%$.

Unless otherwise indicated, the stated percentages are always weight percentages.

The numerical ranges disclosed herein are meant to include any number falling within the ranges and also the lower and upper limits.

Coenzyme Q10

Coenzyme Q10, frequently abbreviated as CoQ10, is a ubiquinone having a side chain of 10 isoprenoid units, also called ubidecarenone, ubiquinone 10 or ubiquinone 50 (Chemical name: 2-[(2E,6E, 10E, 14E, 18E,22E,26E,30E,34E)-3,7,11,15,19,23,27,31,35,39-Decamethyltetraconta-2,6,10,14,18,22,26,30,34,38-decaenyl]-5,6-dimethoxy-3-methylcyclohexa-2,5-diene-1,4-dione, CAS number 303-98-0).

Non-compacted CoQ10, which is the starting material for the process of the present invention is typically produced by microbial fermentation (Lee et al., Cellular factories for coenzyme Q10 production, Microb. Cell Fact., 2017, 16, 39). Furthermore, CoQ10 is widely available from different commercial sources.

Said non-compacted coenzyme Q10 starting material is typically in the form of fine particles, as a powder, namely, it is available as a yellow to orange crystalline powder. It has low melting point, ranging from 48 °C to 52 °C, depending on the reporting source. It is hygroscopic, very adherent, and has poor flowability. Its bulk (or apparent) density is generally less than 0.25 mg/ml, for example about 0.22 mg/ml. Its tapped (or compacted) density is typically less than 0.38 mg/ml, for example about 0.34 mg/ml.

Non-compacted, commercially available coenzyme Q10 used for the preparation of compacted coenzyme Q10, according to the process of the present invention, is typically a powder of fine particle size, with mean particle size generally of less than 800 microns, or less than 700 microns, or less than 600 microns, or less than 500 microns, or less 400 microns, or less than 300 microns, typically the mean particle size is in the range 100-300 microns.

CoQ10 from any source, either manufactured by any suitable method or obtained from any commercial source, can be used as starting material for the process of the present invention, provided that it has sufficient level of purity.

Non-compacted coenzyme Q10 used as starting material, as well as the compacted coenzyme Q10 obtained with the process of the invention, is substantially pure. That means that coenzyme Q10 has substantially no impurities and also that it is compacted alone, that is to say, it is not mixed with any other substance for performing the roller compaction process of the present invention, namely, it is not mixed with any excipient or vehicle.

Therefore, along the present description, as well as in the claims, both non-compacted coenzyme Q10 used as starting material and compacted coenzyme Q10 obtained with the process of the invention are always meant to be substantially pure, which typically means a purity of at least 95%, preferably of at least 96%, more preferably of at least 97%, still more preferably of at least 98%, and still more preferably of at least 98.5%. The purity can advantageously be even higher, of at least 99%, or of at least 99.5%, or about 100%. In one embodiment, the coenzyme Q10, both the starting material and the compacted product, fulfils the specifications of the pharmacopoeia of reference (e.g., 98.0% to 102.0% as stated in European Pharmacopoeia 10.0).

The purity degree of CoQ10 can be determined using methods known in the art, for example, by HPLC, for example, as disclosed in Lunetta et al., J AOAC Int., 2008, 91 (4), 702-8 or in the Ubidecarenone monograph of the European Pharmacopoeia 10.0.

Compaction process

Non-compacted, commercially available coenzyme Q10, as defined above, is used as starting material to prepare compacted coenzyme Q10, according to the process of the present invention.

The compaction process is performed using the roller compaction technology, which is well-known in the pharmaceutical field, namely, for the dry granulation of pharmaceutical powder mixtures. Such technology is widely described in different reference manuals in the art, for example, in R.W. Miller, Roller Compaction Technology, in: Handbook of Pharmaceutical Granulation Technology, Editor D.M. Parikh, Third Edition, Informa Healthcare USA, 2010, Chapter 8, 163-182. Briefly, in the roller compaction process, the powder being compacted is squeezed between two counter-rotating rolls to form a compressed sheet, which is subsequently milled into granules.

Roller compaction machines are well known in the art and are commercially available through many different companies, for example, Eurotab Bonals, Gerteis Maschinen + Processengineering, Freund Corporation, Alexander-Werk, Powtec, Hosokawa Alpine or L.B. Bohle Maschinen und Verfahrenmany, among many others.

As is well-known in the art, the roller compaction machines, or roller compactors, typically comprise a main roller unit connected with a feeding system, for feeding the starting material to be compressed into the roller unit, and are connected to a granulating/milling system, for milling the compacted sheet exiting from the rollers.

The roller compactors available in the market may be characterized according to several parameters, for example, the type of feeding system, the range of compacting forces, the roll speed range, the diameter and the width (also referred to as the length) of the rolls, among others.

Some of these parameters can be adjusted during the compaction process in order to optimize the results, for example, the feed speed, roll speed, roll pressure, roll gap and mill speed.

As is well-known in the art, the feeding system in the roller compactor has the task of feeding the powder to be compacted to the rolls and ensures a uniform flow of material in order to continuously fill the nip between the rolls as they rotate. It can be a gravity feeder, wherein the powder is fed vertically by gravity, or a force feeder, wherein the powder is pushed towards the rolls by one or several screws. Preferably, screw feeding is used in the present invention, and the feeder can be vertical, horizontal or inclined. In one embodiment, the screw feeder is vertical and is situated in the upper part of the equipment, over the rolls. The screw feeder may be a hopper or analogous container containing an endless screw.

The feed speed can be adjusted, preferably to a constant feed speed, for example, by adjusting the rotation speed of the endless screw. Typically, the screw rotation speed is comprised between about 5 and about 70 r.p.m. In one embodiment of the invention, the screw rotation speed is comprised between 10 and 50 r.p.m., preferably is comprised between 10 and 30 r.p.m., more preferably comprised between 12 and 20 r.p.m. and still more preferably is about 15 r.p.m.

Typically, non-compacted coenzyme Q10 raw material is sent for compaction into the screw feeder from another hopper or reservoir containing the product, for example, using vacuum for sucking the powder and sending it to the screw feeder in a controlled way. The sucking of the raw material and its delivery into the screw feeder can be controlled in an automatic way, for example, by placing a probe inside the feeder that activates the feeding of the raw material when necessary.

The roller unit consists of two equal diameter counter rotating rolls through which the powder is passed to get compacted. The two rolls can be mounted in horizontal, vertical or inclined position.

Typically, the roll diameter may range from about 100 to about 450 mm, depending on the roller compactor used. The roll diameter of the roller compactor to be used is not crucial, and all are suitable for performing the process of the current invention. For example, roll diameter of 150-250 mm is particularly suitable.

Typically, the roll width (also referred to as roll length) may range from about 20 to about 120 mm, and any width may be suitable for performing the method of the present invention. The higher the width of the rollers, the higher the amount of coenzyme Q10 that can be processed per unit of time. For example, widths comprised between 50 and 100 mm are suitable.

The roll speed can also be adjusted and typically may range from about 5 r.p.m to about 35 r.p.m. In one embodiment of the invention the roll speed is comprised between 5 and 20 r.p.m., more preferably comprised between 5 and 15 r.p.m., and still more preferably between 7 and 10 r.p.m., for example, is about 7 r.p.m. or is about 8 r.p.m., or is about 9 r.p.m. or is about 10 r.p.m.

Another adjustable parameter is the roll gap, i.e., the distance between the two rolls. It typically ranges from about 0.2 mm to about 2 mm, preferably from about 0.2 mm to about 1.5 mm, more preferably from about 0.3 mm to about 1.0 mm, and still more preferably from about 0.4 mm to about 0.8 mm. In particular embodiments of the invention, the roll gap is selected from about 0.4 mm, about 0.5 mm, about 0.6 mm, about 0.7 mm and about 0.8 mm. In one embodiment, the roll gap is about 0.5 mm.

Generally, one roll is fixed, while the other is movable and exerts pressure on the fixed roll by means of a hydraulic pressure control system. The horizontal pressure/force between rolls can be also adjusted to optimize the properties of the end-product.

The roller compactors available in the market may provide a wide range of compacting forces, generally ranging from about 10 kN to about 200 kN or higher. Typically, the compacting force is comprised between 50 kN and 200 kN, preferably between 60 kN and 150 kN, more preferably between 100 kN and 140 kN and still more preferably comprised between 127 kN and 137 kN.

The roll force applied during the compression may be suitably expressed, for example, as force per unit of roller length. Typically, the force per unit of roller length in the method of the invention may range from about 1 kN/cm to about 30 kN/cm, preferably from about 1.5 kN/cm to about 25 kN/cm, preferably from about 5 kN/cm to about 24 kN/cm, more preferably from about 8 kN/cm to about 21 kN/cm, still more preferably from about 15 kN/cm to about 20 kN/cm, still more preferably from about 16 kN/cm to about 19.5 kN/cm, and still more preferably from about 17 kN/cm to about 19 kN/cm.

The roll surface may be either smooth or non-smooth, i.e., may have rough pattern surface, for example, fluted, grooved or knurled surface. The roll surface of the two rolls may be identical or different.

In one embodiment of the invention, both rolls have smooth surface. In another embodiment of the invention, one roll has a smooth surface and the other has a non-smooth surface, for example, fluted, grooved or knurled surface.

Once coenzyme Q10 has been compacted, after passing through the two counter rotating rollers, it is transformed in a compressed sheet or ribbon, which is subsequently sent to the granulation/milling module, which is typically placed under the compacting rollers. Such granulation/milling module comprises one or more mills which break the compacted ribbon into compacted granules. Typically, after grinding, the granules are sieved through the suitable mesh size to obtain granules of the desired particle size. In this step of the compaction process, the speed of the granulation mill can also be adjusted. This speed is not critical, and is typically comprised in the range of from about 10 r.p.m. to about 500 r.p.m, preferably from about 40 r.p.m. to about 200 r.p.m., more preferably from about 50 r.p.m. to about 100 r.p.m, for example is selected from about 50 r.p.m. about 60 r.p.m., about 70 r.p.m., about 80 r.p.m., about 90 r.p.m., and about 100 r.p.m., more preferably is about 70 r.p.m.

A parameter which has to be controlled during the compression process of coenzyme Q10 is the temperature of the material being compressed.

This temperature is in the range 10-25 °C, preferably in the range 15-25 °C, and more preferably in the range 20-25 °C.

The temperature during compression can be kept under control by using any suitable refrigerating system and typically a temperature sensor. For example, the rollers may advantageously comprise an internal circuit with a circulating refrigerating/heating fluid whose flow can be regulated in order to adjust the temperature to the desired value, and typically comprising a temperature sensor, for example, on the roller surface. The temperature of the roller surface correlates well with the temperature of the product while it is being compressed between the rollers.

In one embodiment, the stated temperature during the compacting process according to the present invention is the temperature of the roller surface.

In one embodiment of the invention, coenzyme Q10 is subjected to roller compaction using the following parameters:

- Roll speed comprised between 5 r.p.m. and 35 r.p.m., preferably between 5 r.p.m. and 20 r.p.m., more preferably between 5 r.p.m. and 15 r.p.m. and still more preferably between 7 r.p.m. and 10 r.p.m., for example, about 7 r.p.m. or about 8 r.p.m. or about 9 r.p.m. or about 10 r.p.m.

- Roll gap comprised between 0.2 mm and 2 mm, preferably between 0.2 mm and 1.5 mm, more preferably between 0.3 mm and 1.0 mm, and still more preferably between 0.4 mm and 0.8 mm, for example, about 0.4 mm or about 0.5 mm, or about 0.6 mm or about 0.7 mm or about 0.8 mm.

- Roll force between the rolls comprised between 50 kN and 200 kN, preferably between 60 kN and 150 kN, more preferably between 100 kN and 140 kN and still more preferably between 127 kN and 137 kN; and/or roll force per unit of roller length comprised between 1 kN/cm and 30 kN/cm, preferably between 1.5 kN/cm and 25 kN/cm, more preferably between 5 kN/cm and 24 kN/cm, still more preferably between 8 kN/cm and 21 kN/cm, still more preferably between 15 kN/cm and 20 kN/cm, still more preferably between 16 kN/cm and 19.5 kN/cm, and still more preferably is comprised between 17 kN/cm and 19 kN/cm;

wherein preferably:

- the screw rotating speed of the feeding system is comprised between 10 r.p.m. and 50 r.p.m., more preferably between 10 r.p.m. and 30 r.p.m., still more preferably between 12 r.p.m. and 20 r.p.m and still more preferably is about 15 r.p.m; and/or

- the speed of the granulation mill is comprised between 40 r.p.m. and 200 r.p.m., more preferably between 50 r.p.m. and 100 r.p.m., for example, is selected from about 60 r.p.m., about 70 r.p.m., about 80 r.p.m., about 90 r.p.m., and about 100 r.p.m., more preferably is about 70 r.p.m.

The compacted coenzyme Q10 obtained with the process according to the present invention has higher density and shows improved flowability compared to the commercial, non-compacted coenzyme Q10. As is well-known in the art, there are several parameters which can be used to assess the flowability of a powder or granulate, namely, the Carr index, Hausner ratio, flow rate and the angle of repose. Some of these parameters are related to the density of the substance, namely, to its bulk (apparent) density and its tapped (compacted) density.

The bulk density, also called apparent density, can be defined as the ratio of the mass to the volume of an untapped powder sample, as "poured". The bulk density is given in grams per millilitre (g/ml) and is typically calculated by "pouring" a known mass of powder into a graduated cylinder.

On the other hand, tapped density, also called compacted density, of a powder is the ratio of the mass of the powder to the volume occupied by the powder after it has been tapped for a defined period of time. Tapped density is measured by first gently introducing a known sample mass into a graduated cylinder and carefully levelling the powder without compacting it. The cylinder is then mechanically tapped by raising the cylinder and allowing it to drop under its own weight using a suitable mechanical tapped density tester that provides a suitable fixed drop distance and nominal drop rate

Bulk density and tapped density can be measured according to the method disclosed in the European Pharmacopoeia section 2.9.34 (*Bulk density and tapped density of powders*), for example, the bulk density may be measured using the PT-SV100 Scott Volumeter and the tapped density may be measured using the PT-TD200 Tap Density Tester (Pharma Test Apparatebau AG, Germany).

The Carr index, also called Carr's index or compressibility index, is calculated according to the following formula:

$$Carr\ index = \frac{Density\,(tapped) - Density\,(bulk)}{Density\,(tapped)} \times 100$$

Free flowing powders have smaller values of the Carr index than poor flowing powders. In general, a Carr index greater than 25 is considered to be an indication of poor flowability, and below 15, of good flowability.

Hausner ratio is also predictive of powder flow, and is calculated according to the following formula:

$$Hausner\ ratio = \frac{Density\,(tapped)}{Density\,(bulk)} \times 100$$

In this case, a Hausner ratio greater than 1.34 is generally considered to be an indication of poor flowability.

The relationship between Carr index and/or Hausner ratio with powder flowability is well recognized in the art, for example, as disclosed by M. E. Aulton, in "Powder flow", chapter 12 of the well-recognized reference book in pharmaceutical technology "Aulton's Pharmaceutics. The design and manufacture of medicines", Fifth Edition 2018, M.E. Aulton and K.M.G. Taylor, Editors, Elsevier Ltd, as summarized in the following table:

| Type of flow | Carr index | Hausner ratio |
|---|---|---|
| Excellent | 1-10 | 1.00-1.11 |
| Good | 11-15 | 1.12-1.18 |
| Fair | 16-20 | 1.19-1.25 |
| Passable | 21-25 | 1.26-1.34 |
| Poor | 26-31 | 1.35-1.45 |
| Very poor | 32-37 | 1.46-1.59 |
| Very, very poor | >37 | >1.59 |

The "angle of repose" is another parameter for indirectly quantifying powder flowability. It is the angle at which a material will rest on a stationary heap, specifically, is the angle formed by the heap slope and the horizontal plane. It may be calculated by different methods, commonly, just pouring the material on a flat surface. It also can be calculated as "drained angle of repose", as the angle measured on the internal conical face of a material which has been formed when drained from an orifice of a flat-bottomed container to the horizontal. A dynamic angle of repose can also be used, which is the angle to the horizontal of the free surface formed in a relatively slowly rotating drum (Aulton, *op. cit.,* pg. 197).

In this case, the lower the value of the angle of repose, the higher the flowability of the material. It is generally considered that an angle of repose higher than 45 correlates with poor flowability, whereas an angle of repose under 30 correlates with excellent flowability.

As disclosed in Example 2, the compacted coenzyme Q10 obtained according to the process of the present invention has excellent flowability, as shown by the values of the Carr index, Hausner ratio and the angle of repose.

In particular, the method of the invention allows obtaining compacted CoQ10 having a Carr index lower than 15, preferably lower than 13, more preferably lower than 11, still more preferably equal to or lower than 10, for example comprised between 5 and 10.

Analogously, the method of the invention allows obtaining compacted CoQ10 having a Hausner ratio lower than 1.25, more preferably lower than 1.20, still more preferably lower than 1.18, still more preferably lower than 1.14, still more preferably lower than 1.12, still more preferably equal to or lower than 1.11, namely, comprised between 1.00 and 1.11.

Non-compacted, commercially available coenzyme Q10, when subjected to the process according to the present invention, undergoes a significant increase of its density; typically, the bulk density increases in more than 100%, from a bulk density of about 0.2 g/ml of the starting non-compacted CoQ10 to a bulk density of more than 0.40 g/ml, preferably more than 0.45 g/ml and more preferably of more than 0.50 g/ml of the compacted CoQ10. Typically, the bulk density of the compacted CoQ10 is in the range 0.45-0.60 g/ml, preferably in the range 0.50-0.55 g/ml.

Additionally, the authors of the present invention assessed that controlling the temperature of the product during the compression process is important for obtaining optimal compacted CoQ10 particles. In particular, they found out that by keeping the temperature low, namely, not higher than 25 °C, the compressed coenzyme Q10 particles obtained have outstanding flowability, particularly, they reach values of the angle of repose as low as less than 20 degrees, or less than 15 degrees, typically of about 10 degrees.

This is surprising, because in the prior art (EP-A-2415467) it is stated that the temperature of CoQ10 during the compression must be in the range 35-52 °C, and it is specifically stated therein that when the product temperature is lower than 35 °C a satisfactory compression molded product is not obtainable. Furthermore, it is stated in the cited prior art that for obtaining the best flowable product, having a low angle of repose of about 7 to 18 degrees, an additional step of heat treatment of the compacted CoQ10 is required, at a temperature in the range 30-52 °C.

The authors of the present invention discovered that, surprisingly, contrary to what is taught in the prior art, by using temperatures much lower than 35 °C, i.e. in the range 10-25 °C and, preferably, in the range 15-25 °C or 20-25 °C, as disclosed above, not only a satisfactory compressed product is indeed obtained, but it has even better properties, in particular, lower value of the angle of repose. Indeed, in the method disclosed in the prior art, such low values of the angle of repose could only be obtained by performing an additional heating step of the particles after the compression. Conversely, according to the method of the present invention, the particles directly obtained after the compression at low temperature, already have good flowability parameters, including an angle of repose as low as about 10°.

The compacted CoQ10 obtained with the process of the present invention is advantageous for manufacturing pharmaceutical, dietary or cosmetic compositions due to its improved rheological properties, so it flows easily and does not stick to the walls of the containers during its handling and during the manufacturing process of said compositions, avoiding the need, for example, of using shaking or vibration. Furthermore, its improved compressibility and higher density make compacted CoQ10 particularly suitable as pharmaceutical, dietary or cosmetic ingredient.

Therefore, the compacted coenzyme Q10 which is obtained by the process of the present invention, as disclosed above, remarkably, despite its known instability, after the compaction process of the present invention, coenzyme Q10 maintains its chemical integrity, so there is neither loss in purity nor increase in impurity occurrence. Therefore, the compaction process according to the present invention enables the preparation of substantially pure compacted CoQ10, with improved flowability characteristics, which has substantially the same purity degree as the starting material. The compacted CoQ10 obtained with the process of the invention is also substantially pure as defined above for the non-compacted CoQ10 starting material, and the purity can be determined in the same manner, typically by HPLC.

The size of the particles of the obtained compacted coenzyme Q10 can be adjusted, for example, by milling, if necessary, and sieving, using a sieve of suitable mesh size to obtain particles of the desired size, namely, under a specific size determined by the sieve opening. Typically, the mesh size of the sieve is adjusted to obtain particles of less than 2000 microns, preferably less than 1800 microns, preferably less than 1600 microns, more preferably less than 1400 microns, still more preferably less than 1200 microns, still more preferably less than 1000 microns, still more preferably less than 900 microns, and still more preferably less than 800 microns. The particle size, for example, is typically comprised between 400 and 2000 microns, preferably comprised between 400 and 1000 microns, and more preferably comprised between 500 and 800 microns.

As discussed above, the compacted coenzyme Q10 obtained with the process of the present invention, has improved flowability and higher density, and can be advantageously used for the preparation of pharmaceutical compositions.

Hence, the improved flowability is an advantage for the manufacturing of pharmaceutical compositions, as the product flows more easily, does not stick to the walls of vessels or blenders, or to the punches and dies of tableting machines, for example, therefore avoiding product loses and improving the equipment maintenance. Furthermore, the improved flowability of the coenzyme Q10 obtained by the process of the invention is also an advantage for preparing powder pre-mixtures with excipients, for example, for manufacturing tablets or capsules, avoiding segregation of the components and providing better homogeneity of the blend.

Furthermore, the improved compressibility (related to its low Carr index) of compacted coenzyme Q10 particles obtained by the process of the invention enables the preparation of tablets of higher hardness by direct compression.

On the other hand, the higher density of compacted coenzyme Q10 prepared according to the process of the present invention enables a reduction of the volume of the final dosage forms, particularly, oral dosage forms such as capsules or tablets, for a given active ingredient strength.

Next, some examples are provided with the purpose of illustrating the invention, but not limiting it.

Examples

Example 1.- Preparation of compacted coenzyme Q10 according to the invention

For the preparation of compacted high-density coenzyme Q10, commercially available coenzyme Q10 (Inner Mongolia Kingdomway Pharmaceutical Ltd) was used. The coenzyme Q10 raw material was a yellow-orange crystalline powder, of bulk density 0.22 g/ml and particle size of less than 800 microns.

The purity of coenzyme Q10 before and after the compaction process was assessed by means of HPLC. The HPLC specifications used were as follows:

| | |
|---|---|
| Equipment: | 1260 Infinity LC (Agilent Technologies). |
| Column: | ACE3 C18 PFP (150 x 4.6 mm) |
| Flow rate: | 1 ml/min |
| Injection volume: | 10 $\mu$l |
| Detector: | 275 nm |
| Run time: | 10 minutes |
| Solvent: | ethanol:methanol (20:80). Isocratic elution |
| Retention time: | 5.25 |

Both the original coenzyme Q10 and the compacted coenzyme Q10 obtained after the compaction process had chemical purity greater than 99%, determined by HPLC.

For the compaction process, a roller compactor equipment was used (Bonals BC-150/75V), with vacuum system for feeding the raw material into the screw feeding system and with a refrigerating system. The roller compactor had one roll with smooth surface and one roll with knurled surface. The dimensions of both rolls were 10.5 cm diameter and 7.4 cm width.

The roll speed in the roller compactor was about 7-8 r.p.m. Screw feeding speed was 15 r.p.m., roll force was set to 17.2-18.6 kN and the roll gap was fixed to 0.5 mm.

The roller compactor used allowed a continuous control of the temperature of the roll surface and had an internal refrigeration circuit, with a circulating refrigeration fluid whose flow could be regulated in order to adjust said temperature to the desired value. The temperature was kept in the range 20-25 °C during the compression process.

The compactor was connected to a rotor granulator for milling the outcoming compacted plaque. The granulation speed was fixed to 70 r.p.m. and the obtained granulate was passed through a sieve of 800 micron opening.

The compacted coenzyme Q10 obtained had a bulk density of 0.52 g/ml and 100% of the particles had a size of less than 800 micron.

Example 2.- Flowability parameters of the compacted coenzyme Q10 prepared in Example 1

The flowability parameters of non-compacted and compacted CoQ10 are compared in the following table:

| Property | Non-compacted CoQ10 (starting material) | Compacted CoQ10 (Example 1) |
|---|---|---|
| Aspect | Orange powder with damp, agglomerated appearance, hygroscopic | Orange granules without agglomerates, highly flowable and with dry appearance |

(continued)

| Property | Non-compacted CoQ10 (starting material) | Compacted CoQ10 (Example 1) |
|---|---|---|
| Bulk density | 0.22 g/ml | 0.52 g/ml |
| Tapped density | 0.34 g/ml | 0.57 g/ml |
| Compressibility (Carr Index) | 35.3% | 8.78% |
| Hausner ratio | 1.55 | 1.10 |
| Flow rate | Slow, the material sticks to the walls | Three times faster than for uncompacted CoQ10. The material does not stick to the walls |
| Angle of repose | 45° | 10° |

The bulk density was measured using a PT-SV100 Scott Volumeter (Pharma Test) and the tapped density was measured using a PT-TD200 Tap Density Tester (Pharma Test).

The angle of repose was determined by measuring the radius and the height of the cone formed after pouring the powder, using the formula $\tan^{-1}$ (height/radius).

Figure 1 shows a picture of non-compacted CoQ10 starting material and Figure 2 shows a picture of compacted CoQ10 after the process of the invention. It can be observed that the non-compacted material has damp and agglomerated appearance, whereas the compacted product has dry, non-agglomerated, flowable appearance.

Example 3.- Preparation of capsules with the compacted coenzyme Q10 of Example 1

Coenzyme Q10 hard gelatin capsules of 100 mg strength were prepared using the ingredients listed in the following table:

| Ingredient | Amount per capsule |
|---|---|
| Compacted CoQ10 (Example 1) | 100 mg |
| Maize starch (diluent) | 200 mg |
| Magnesium stearate (lubricant) | 10 mg |

All ingredients were added to a Turbula mixer, blended during 15 minutes, and filled into capsules of 00 size.

The capsules were easily prepared, it was observed that the compacted CoQ10 used had good flowability and did not adhere to the walls of vessels and mixers. Furthermore, the powder mixture was easily blended and good homogeneity was easily achieved, without segregation of the ingredients.

Example 4.- Preparation of tablets by direct compression with the compacted coenzyme Q10 of Example 1

Coenzyme Q10 tablets of 30 mg strength were prepared using the ingredients listed in the following table:

| Ingredient | Amount per tablet |
|---|---|
| Compacted CoQ10 (Example 1) | 30 mg |
| Calcium phosphate (diluent) | 75 mg |
| Microcrystalline cellulose (diluent) | 100 mg |
| Magnesium stearate (lubricant) | 2 mg |
| Silica (glidant) | 2 mg |

All ingredients were mixed in Turbula mixer during about 15 minutes and compressed into tablets.

It was also observed that the compacted CoQ10 used had good flowability and did not adhere either to the walls of vessels and mixers or to the punches and dies of the tableting machine. The powder mixture was easily blended and had good homogeneity, without segregation of the ingredients.

**Claims**

1. A process for preparing compacted coenzyme Q10 comprising roller compaction of coenzyme Q10 at a temperature comprised between 10 °C and 25 °C.

2. Process according to claim 1, **characterized in that** the temperature is comprised between 15 °C and 25 °C.

3. Process according to claim 1, **characterized in that** the temperature is comprised between 20 °C and 25 °C.

4. Process according to claim 1 or 3, **characterized in that** the roll speed is adjusted to a value comprised between 5 r.p.m. and 20 r.p.m., preferably comprised between 5 r.p.m. and 15 r.p.m.

5. Process according to any one of claims 1 to 4, **characterized in that** the gap between the rollers is adjusted to a value comprised between 0.2 mm and 2 mm, preferably comprised between 0.3 mm and 1.0 mm, and more preferably comprised between 0.4 mm and 0.8 mm.

6. Process according to any one of claims 1 to 5, **characterized in that** the roll force is adjusted to a value comprised between 60 kN and 150 kN, preferably between 100 kN and 140 kN and more preferably between 127 kN and 137 kN.

7. Process according to any one of claims 1 to 5, **characterized in that** the roll force per unit of roller length is adjusted to a value comprised between 8 kN/cm and 21 kN/cm, preferably comprised between 15 kN/cm and 20 kN/cm, more preferably comprised between 16 kN/cm and 19.5 kN/cm, and still more preferably comprised between 17 kN/cm and 19 kN/cm.

**Patentansprüche**

1. Verfahren zur Herstellung von verdichtetem Coenzym Q10 umfassend die Walzenverdichtung von Coenzym Q10 bei einer Temperatur, welche zwischen 10 °C und 25 °C liegt.

2. Verfahren nach Anspruch 1, **dadurch gekennzeichnet, dass** die Temperatur zwischen 15 °C und 25 °C liegt.

3. Verfahren nach Anspruch 1, **dadurch gekennzeichnet, dass** die Temperatur zwischen 20 °C und 25 °C liegt.

4. Verfahren nach Anspruch 1 oder 3, **dadurch gekennzeichnet, dass** die Walzengeschwindigkeit auf einen Wert, welcher zwischen 5 UPM und 20 UPM liegt, vorzugsweise zwischen 5 UPM und 15 UPM liegt, eingestellt wird.

5. Verfahren nach einem der Ansprüche 1 bis 4, **dadurch gekennzeichnet, dass** der Spalt zwischen den Walzen auf einen Wert, welcher zwischen 0,2 mm und 2 mm liegt, vorzugsweise zwischen 0,3 mm und 1,0 mm liegt, und weiter vorzugsweise zwischen 0,4 mm und 0,8 mm liegt, eingestellt wird.

6. Verfahren nach einem der Ansprüche 1 bis 5, **dadurch gekennzeichnet, dass** die Walzkraft auf einen Wert, welcher zwischen 60 kN und 150 kN, vorzugsweise zwischen 100 kN und 140 kN und weiter vorzugsweise zwischen 127 kN und 137 kN liegt, eingestellt wird.

7. Verfahren nach einem der Ansprüche 1 bis 5, **dadurch gekennzeichnet, dass** die Walzkraft pro Einheit Walzenlänge auf einen Wert, welcher zwischen 8 kN/cm und 21 kN/cm liegt, vorzugsweise zwischen 15 kN/cm und 20 kN/cm liegt, weiter vorzugsweise zwischen 16 kN/cm und 19,5 kN/cm liegt, und noch weiter vorzugsweise zwischen 17 kN/cm und 19 kN/cm liegt, eingestellt wird.

**Revendications**

1. Procédé de préparation de coenzyme Q10 compactée comprenant un compactage aux rouleaux de coenzyme Q10 à une température comprise entre 10 °C et 25 °C.

**2.** Procédé selon la revendication 1, **caractérisé en ce que** la température est comprise entre 15 °C et 25 °C.

**3.** Procédé selon la revendication 1, **caractérisé en ce que** la température est comprise entre 20 °C et 25 °C.

**4.** Procédé selon la revendication 1 ou 3, **caractérisé en ce que** la vitesse de rouleau est ajustée à une valeur comprise entre 5 tours par minute et 20 tours par minute, de préférence comprise entre 5 tours par minute et 15 tours par minute

**5.** Procédé selon l'une quelconque des revendications 1 à 4, **caractérisé en ce que** l'écart entre les rouleaux est ajusté à une valeur comprise entre 0,2 mm et 2 mm, de préférence comprise entre 0,3 mm et 1,0 mm, et plus de préférence comprise entre 0,4 mm et 0,8 mm.

**6.** Procédé selon l'une quelconque des revendications 1 à 5, **caractérisé en ce que** la force de rouleau est ajustée à une valeur comprise entre 60 kN et 150 kN, de préférence entre 100 kN et 140 kN et plus de préférence entre 127 kN et 137 kN.

**7.** Procédé selon l'une quelconque des revendications 1 à 5, **caractérisé en ce que** la force de rouleau par unité de longueur de rouleau est ajustée à une valeur comprise entre 8 kN/cm et 21 kN/cm, de préférence comprise entre 15 kN/cm et 20 kN/cm, plus de préférence comprise entre 16 kN/cm et 19,5 kN/cm, et encore plus de préférence comprise entre 17 kN/cm et 19 kN/cm.

FIGURE 1

FIGURE 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100004473 A
- EP 2415467 A

**Non-patent literature cited in the description**

- **GARRIDO-MARAVER et al.** Coenzyme Q10 Therapy. *Mol. Syndromol.*, 2014, 187-197
- **HOPPE et al.** Coenzyme Q10, a cutaneous antioxidant and energizer. *BioFactors*, 1999, vol. 9, 371-378
- **BEG et al.** Bioavailability enhancement of Coenzyme Q10: an extensive review of patents. *Recent patents on Drug Delivery & Formulation*, 2010, vol. 4, 245-257
- **ARENAS-JAL et al.** Coenzyme Q10 supplementation: Efficacy, safety, and formulation challenges. *Compr. Rev. Food Sci. Food Saf.*, 2020, vol. 19, 574-594
- **LEE et al.** Cellular factories for coenzyme Q10 production. *Microb. Cell Fact.*, 2017, vol. 16, 39
- **LUNETTA et al.** *J AOAC Int.*, 2008, vol. 91 (4), 702-8
- Handbook of Pharmaceutical Granulation Technology. **R.W. MILLER**. Roller Compaction Technology. Informa Healthcare USA, 2010, 163-182
- Aulton's Pharmaceutics. The design and manufacture of medicines. Elsevier Ltd, 2018